# EUROPEAN PATENT APPLICATION

(11) **EP 4 120 191 A2**
(43) Date of publication of application: **18.01.2023**
(21) Application number: 22179762.4
(22) Date of filing: 17.06.2022
(51) Int. Cl.: G06T 7/11

(54) **TOOTH SEGMENTATION APPARATUS AND METHOD FOR TOOTH IMAGE**

(30) Priority: 22.06.2021 KR 20210080998
(71) Applicant: 3D Industrial Imaging Co., Ltd., Seoul 08826 (KR)
(72) Inventor: KIM, Taehyung, Seoul 06291 (KR); JOE, Haeyoung, Seoul 08828, (KR); KIM, Kyunyun, Gyeonggi-do 13909, (KR)
(74) Representative: Germain Maureau

(57) **Abstract**

The tooth segmentation apparatus detects a boundary box of each tooth from dental scan data in a form of a mesh, sets a boundary condition from the boundary box of each tooth, and segments a tooth region of each tooth in the dental scan data based on the boundary condition of each tooth.

## Description

### CROSS-REFERENCE TO RELATED APPLICATION

This application claims priority to and the benefit of Korean Patent Application No. 10-2021-0080998 filed in the Korean Intellectual Property Office on June 22, 2021, the entire contents of which are incorporated herein by reference.

### BACKGROUND

### (a) Field

The present disclosure relates to a tooth segmentation apparatus and method for a tooth image.

### (b) Description of the Related Art

When a dental implant or orthodontic treatment is performed, it is necessary to specify positions and types of teeth. Tooth numbers can be used as clinical information to specify the positions and types of teeth.

In order to assign a tooth number to each tooth in a teeth image such as teeth scan data, it is necessary to find a boundary between the teeth and segment the teeth. A method for calculating the boundary between teeth by solving the Laplace equation based on a user input may be used. In this case, the user creates a mark on the tooth and a large circle including the mark through two clicks per tooth.

Since the two clicks are used per tooth, 56 clicks are required to segment all 28 upper and lower teeth. This can lead to increased working time and increased user fatigue.

### SUMMARY

Some embodiments of the present invention may provide a tooth segmentation device and method capable of segmenting a tooth region with no or minimal user input.

According to an embodiment of the present invention, there is provided a tooth segmentation apparatus including a memory configured to store one or more instructions and a processor configured to execute the one or more instructions. By executing the one or more instructions, the processor may detect a boundary box of each tooth from dental scan data in a form of a mesh, set a boundary condition from the boundary box of each tooth, and segment a tooth region of each tooth in the dental scan data based on the boundary condition of each tooth.

In some embodiments, the processor may detect the bounding box of each tooth from the dental scan data based on an object detection model of an artificial neural network.

In some embodiments, the processor may derive a first vertex of the mesh corresponding to a center of the bounding box and a plurality of second vertices of the mesh respectively corresponding to a plurality of vertices of the bounding box, find a length of a predetermined shorted path among a plurality of shortest paths from each of the plurality of second vertices to the first vertex, and assign values of the boundary condition to a plurality of third vertices of the mesh corresponding to the length of the predetermined shortest path from the first vertex.

In some embodiments, the predetermined shortest path may be a longest shortest path among the plurality of shortest paths.

In some embodiments, the processor may segment the tooth region based on a Laplace equation using the values of the boundary condition as an input.

In some embodiments, the processor may generate a plurality of tooth regions of a plurality of teeth by segmenting the tooth region of each tooth in the dental scan data, provide an interface configured to check segmentation of the tooth region of each tooth, and receive an operation for correction of a tooth region requiring correction among the plurality of tooth regions.

In some embodiments, the processor may assign a corresponding color to the tooth region of each tooth, and receive the operation for correction through coloring of the tooth region requiring correction.

In some embodiments, the processor may generate a plurality of tooth regions of a plurality of teeth by segmenting the tooth region of each tooth in the dental scan data, display the plurality of tooth regions, receive a designation for a number of at least one tooth among the plurality of teeth from a user, and automatically numbering remaining teeth among the plurality of teeth based on the number of the at least one tooth.

In some embodiments, the processor may automatically designate the numbers of the remaining teeth according to a clinically used numbering system.

In some embodiments, the processor may provide an interface configured to check a number of each tooth, receive an operation for number correction of a tooth whose number requires correction among the plurality of teeth through the interface, and automatically correct a number of a portion of the plurality of teeth based on the number correction.

In some embodiments, the processor may select the portion of plurality of teeth according to a clinically used numbering system.

According to another embodiment of the present invention, a tooth segmentation method performed by a computing device is provided. The tooth segmentation method includes receiving dental scan data in a form of a mesh, detecting a boundary box of each tooth from the dental scan data, setting a boundary condition from the boundary box of each tooth, and segmenting a tooth region of each tooth in the dental scan data based on the boundary condition of each tooth.

In some embodiments, detecting the bounding box may include detecting the bounding box of each tooth from the dental scan data based on an object detection model of an artificial neural network.

In some embodiments, setting the boundary condition may include deriving a first vertex of the mesh corresponding to a center of the bounding box and a plurality of second vertices of the mesh respectively corresponding to a plurality of vertices of the bounding box, finding a length of a predetermined shorted path among a plurality of shortest paths from each of the plurality of second vertices to the first vertex, and assigning values of the boundary condition to a plurality of third vertices of the mesh corresponding to the length of the predetermined shortest path from the first vertex.

In some embodiments, the tooth segmentation method may further include generating a plurality of tooth regions of a plurality of teeth by segmenting the tooth region of each tooth in the dental scan data, providing an interface configured to check segmentation of the tooth region of each tooth, and receiving an operation for correction of a tooth region requiring correction among the plurality of tooth regions.

In some embodiments, the tooth segmentation method may further include generating a plurality of tooth regions of a plurality of teeth by segmenting the tooth region of each tooth in the dental scan data, displaying the plurality of tooth regions, receiving a designation for a number of at least one tooth among the plurality of teeth from a user, and automatically numbering remaining teeth among the plurality of teeth based on the number of the at least one tooth.

In some embodiments, automatically designating numbering the remaining teeth may include automatically numbering the remaining teeth according to a clinically used numbering system.

In some embodiments, the tooth segmentation method may further include providing an interface configured to check a number of each tooth, receiving an operation for number correction of a tooth whose number requires correction among the plurality of teeth through the interface, and automatically correcting a number of a portion of the plurality of teeth based on the number correction.

According to yet another embodiment of the present invention, a computer program executed by a computing device and stored in a non-transitory recording medium is provided. The computer program may cause the computing device to execute receiving dental scan data in a form of a mesh, detecting a boundary box of each tooth from the dental scan data, setting a boundary condition from the boundary box of each tooth, and segmenting a tooth region of each tooth in the dental scan data based on the boundary condition of each tooth.

In some embodiments, the computer program may cause the computing device to execute generating a plurality of tooth regions of a plurality of teeth by segmenting the tooth region of each tooth in the dental scan data, displaying the plurality of tooth regions, receiving a designation for a number of at least one tooth among the plurality of teeth from a user, and automatically numbering remaining teeth among the plurality of teeth based on the number of the at least one tooth.

### BRIEF DESCRIPTION OF THE DRAWINGS

FIG. 1 is a block diagram showing an example of a tooth segmentation apparatus according to an embodiment of the present invention.
FIG. 2 is a flowchart showing an example of a tooth segmentation method according to an embodiment of the present invention.
FIG. 3 is a drawing showing an example of a boundary box of a tooth in a tooth segmentation method according to an embodiment of the present invention.
FIG. 4 is a drawing showing an example of a vertex of a mesh in a tooth segmentation method according to an embodiment of the present invention.
FIG. 5 is a drawing showing an example of a segmented tooth region in a tooth segmentation method according to an embodiment of the present invention.
FIG. 6 is a drawing showing an example of a tooth region requiring correction in a tooth segmentation method according to an embodiment of the present invention.
FIG. 7 is a flowchart showing an example of a tooth numbering method in a tooth segmentation method according to an embodiment of the present invention.
FIG. 8 is a drawing showing an example of a number designated by a user in a tooth numbering method according to an embodiment of the present invention.
FIG. 9 is a drawing showing an example of numbers automatically assigned in a tooth numbering method according to an embodiment of the present invention.
FIG. 10 is a drawing showing an example of a number that needs correction in a tooth numbering method according to an embodiment of the present invention.
FIG. 11 is a drawing showing an example of a modified number in a tooth numbering method according to an embodiment of the present invention.
FIG. 12 is a drawing showing an example of a computing device according to an embodiment of the present invention.

### DETAILED DESCRIPTION OF THE EMBODIMENTS

In the following detailed description, only certain embodiments of the present invention have been shown and described, simply by way of illustration. As those skilled in the art would realize, the described embodiments may be modified in various different ways, all without departing from the spirit or scope of the present invention. Accordingly, the drawings and description are to be regarded as illustrative in nature and not restrictive. Like reference numerals designate like elements throughout the specification.

As used herein, a singular form may be intended to include a plural form as well, unless the explicit expression such as "one" or "single" is used.

In flowcharts described with reference to the drawings, the order of operations or steps may be changed, several operations or steps may be merged, a certain operation or step may be divided, and a specific operation or step may not be performed.

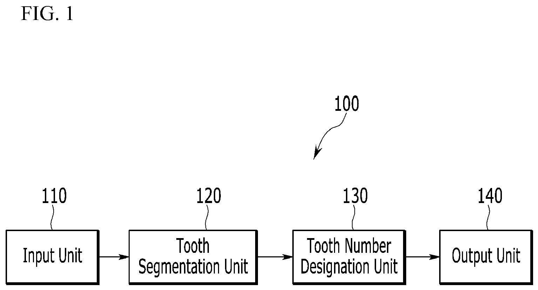

FIG. 1 is a block diagram showing an example of a tooth segmentation apparatus according to an embodiment of the present invention.

Referring to FIG. 1, a tooth segmentation apparatus includes an input unit 110, a tooth segmentation unit 120, and an output unit 140.

The input unit 110 receives dental scan data. In some embodiments, the dental scan data may include dental scan data in the form of a mesh. In some embodiments, the dental scan data may be obtained from 3D scan data obtained by scanning a person's oral cavity with an oral scanner. The dental scan data may include data representing a surface of a three-dimensional shape of the oral cavity including teeth in the form of a mesh.

The tooth segmentation unit 120 detects a boundary box of each tooth from the dental scan data, sets a boundary condition from the boundary box of each tooth, and divides a tooth region of each tooth in the dental scan data based on the boundary condition of each tooth.

In some embodiments, the tooth segmentation apparatus may further include a tooth number designation unit 130. The tooth number designation unit 130 may receive a designation for the number of at least one tooth among a plurality of teeth from a user, and automatically designate the numbers of remaining teeth based on the designated number.

The output unit 140 outputs a tooth region (e.g., mesh) and the tooth number of an individual teeth which has been finally segmented. In some embodiments, the output unit 140 may include a display device and display the tooth region and the tooth number on the display device.

In some embodiments, the tooth segmentation apparatus may be implemented by a computing device.

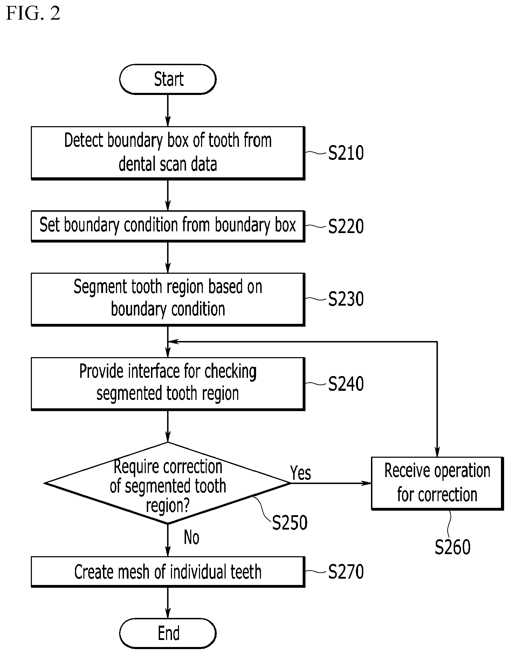

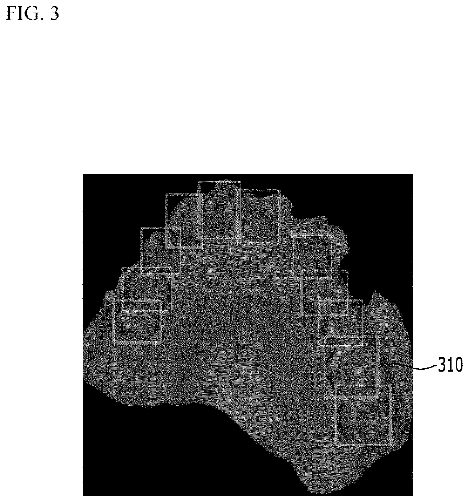

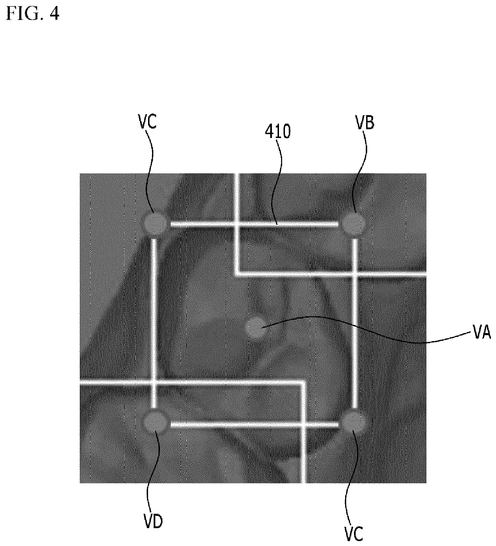

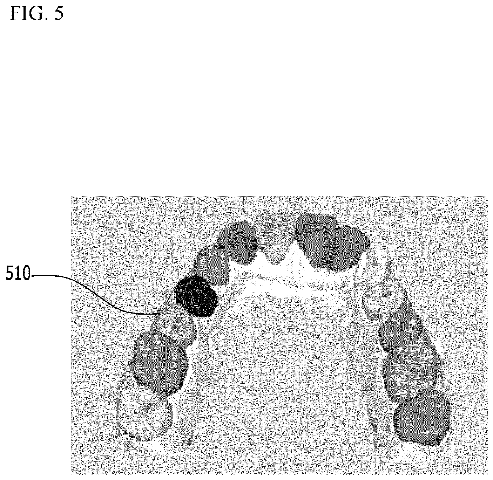

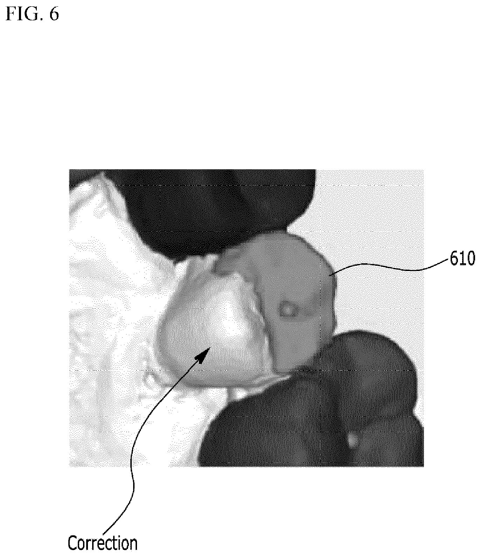

FIG. 2 is a flowchart showing an example of a tooth segmentation method according to an embodiment of the present invention, FIG. 3 is a drawing showing an example of a boundary box of a tooth in a tooth segmentation method according to an embodiment of the present invention, and FIG. 4 is a drawing showing an example of a vertex of a mesh in a tooth segmentation method according to an embodiment of the present invention. FIG. 5 is a drawing showing an example of a segmented tooth region in a tooth segmentation method according to an embodiment of the present invention, and FIG. 6 is a drawing showing an example of a tooth region requiring correction in a tooth segmentation method according to an embodiment of the present invention.

Referring to FIG. 2, a tooth segmentation apparatus, for example, a tooth segmentation unit of the tooth segmentation apparatus detects a boundary box of a tooth from dental scan data in the form of a mesh at S210. In some embodiments, the tooth segmentation unit may receive the dental scan data including upper jaw scan data in the form of a mesh obtained by scanning an upper jaw and lower jaw scan data in the form of a mesh obtained by scanning a lower jaw. In some embodiments, the tooth segmentation unit may obtain a rendering screen of a tooth mesh and extract the bounding box of the tooth on a two-dimensional image. The tooth segmentation unit may extract the boundary box corresponding to an object (i.e., tooth) by inputting the dental scan data to an object detection model of an artificial neural network (also referred to as a "neural network"). The boundary box may be a box that roughly indicates a boundary of the detected object (tooth) in two dimensions. In some embodiments, the object detection model may use various types of machine learning models, for example, YOLO, Detectron, and the like. As shown in FIG. 3, the tooth segmentation unit may extract the boundary box 310 corresponding to each tooth through the object detection model. In some embodiments, the object detection model may be a model which has been trained by using a training data set.

Next, the tooth segmentation unit sets a boundary condition of each tooth from the extracted boundary box of the corresponding tooth at S220, and segments a tooth region of each tooth in the dental scan data based on the boundary condition of each tooth at S230. The tooth segmentation unit may generate a plurality of tooth regions of a plurality of teeth by segmenting the tooth region of each tooth in the dental scan data.

In some embodiments, the boundary condition may be a boundary condition for solving the Laplace equation in the mesh, and may be set by assigning a specific value to a selected point on the mesh. In some embodiments, as shown in FIG. 4, the tooth segmentation unit may derive a vertex VA of the mesh corresponding to a center of the bounding box 410 and vertices VB, VC, VD, and VE of the mesh corresponding to four vertices of the bounding box 410. The tooth segmentation unit may assign a predetermined value (e.g., '1') to the central vertex VA. The tooth segmentation unit may calculate the lengths of the shortest paths from the vertices VB, VC, VD, and VE to the vertex VA along edges of the mesh, respectively, and find the longest length (n) among the shortest paths. The tooth segmentation unit may assign a value (e.g., '0') of the boundary condition to vertices corresponding to the longest length (n) from the central vertex VA, that is, a plurality of vertices corresponding to the n-ring neighborhood from the central vertex VA. In this way, the tooth segmentation unit may set a circle having the central vertex VA as a center and the longest length (n) as a radius. In some embodiments, the tooth segmentation unit may obtain a three-dimensional point through ray-casting from the center of the boundary box 410 to the mesh, and designate a vertex of the mesh closest to the obtained point as the vertex VA. In addition, the tooth segmentation unit may obtain three-dimensional points through the ray-casting from the four vertices of the bounding box to the mesh, and designate vertices of the mesh closest to the obtained points as the vertices VB, VC, VD, and VE, respectively.

The tooth segmentation unit may set a boundary of each tooth (i.e., a boundary between the corresponding tooth and an adjacent tooth and a boundary between the corresponding tooth and the gum) based on the value of the boundary condition of the corresponding tooth, and segment each tooth from the other teeth and the gum at S230. In some embodiments, for each tooth, the tooth segmentation unit may solve the Laplacian equation using the value of the boundary condition as an input to find the boundary of the corresponding tooth as shown in FIG. 5. For example, the boundary 510 shown in FIG. 5 may be found from the bounding box 410 shown in FIG. 4. For example, the tooth segmentation unit may build the Laplace equation on the mesh surface based on the value of '1' assigned to the central vertex VA, the values of '0' assigned to the n-ring vertices, and coefficient specified for the edges connecting the vertices and solve the Laplace equation. Then, vertices included within the boundary formed by the n-ring vertices may have real values between 0 and 1. The tooth segmentation unit may set the boundary of the tooth by selecting an appropriate real value between 0 and 1 and calculating an isoline on the mesh surface. The tooth segmentation unit may automatically select the real value used to calculate the isoline based on the mesh information.

In some embodiments, the tooth segment may assign an arbitrary color to a region included within the boundary of the tooth, i.e., a region of the individual tooth. In some embodiments, colors assigned to the adjacent teeth may be different from each other.

In some embodiments, the tooth segmentation unit may provide an interface for checking the segmented tooth region at S240, and may, when there is a tooth region that needs to be corrected because the boundary is set incorrectly at S250, receive a user's operation for correcting the tooth region through the interface at S260. The user may check whether there is no error in the tooth region of each tooth through the interface. When there is a tooth region 610 whose boundary is incorrectly set as shown in FIG. 6, the user may input an operation for correcting the boundary of the tooth region 610. In some embodiments, the interface for checking the segmented tooth region may be provided as a screen where the user can directly modify the tooth region. In this case, for example, the user may select a region to be modified and then select a face of the mesh to be included in the tooth region of the corresponding tooth through coloring. For example, the user's coloring may be performed by using a mesh-coloring technique.

When there is no tooth region whose boundary is incorrectly set at S250, the tooth segmentation unit may create a mesh of the individual teeth at S270. In some embodiments, when there is no tooth region whose boundary is incorrectly set at S250, the tooth segmentation unit may receive an input of a user's operation to confirm that there is no tooth region whose boundary is incorrectly set.

According to the above-described embodiments, the user's work can be minimized because the user's input for setting the boundaries of the teeth is not required. Further, since the tooth region can be segmented with the minimum input of the user, the working time (i.e., computing time) can be reduced. Furthermore, providing the interface capable of correcting the incorrectly segmented tooth region can prevent the tooth region from being incorrectly segmented by

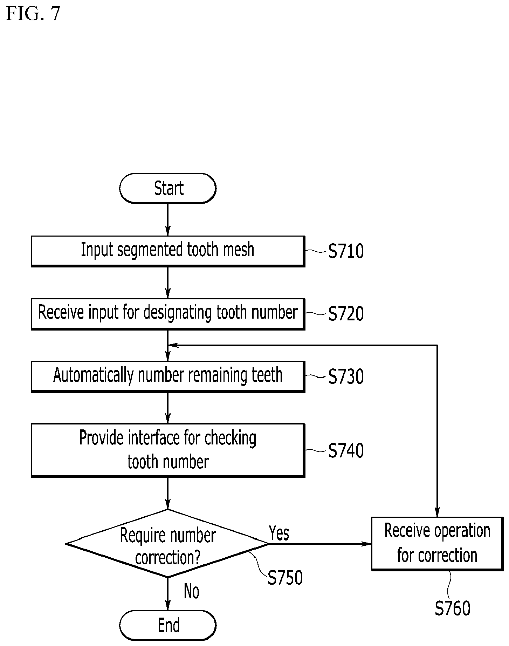

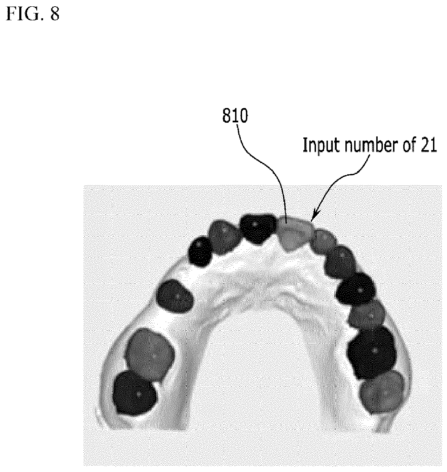

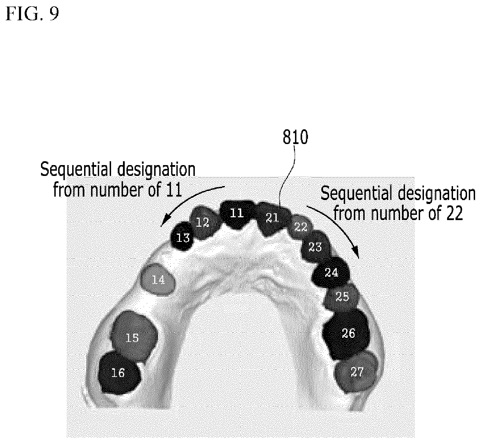

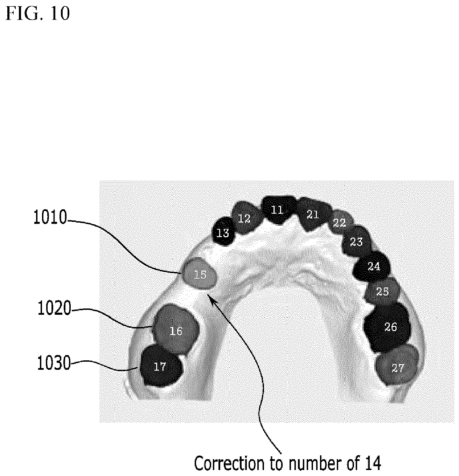

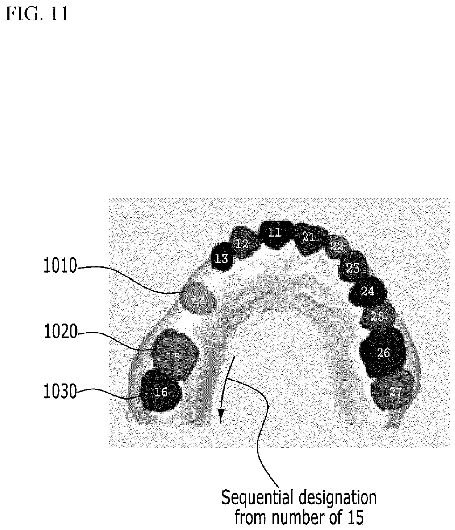

FIG. 7 is a flowchart showing an example of a tooth numbering method in a tooth segmentation method according to an embodiment of the present invention, FIG. 8 is a drawing showing an example of a number designated by a user in a tooth numbering method according to an embodiment of the present invention, and FIG. 9 is a drawing showing an example of numbers automatically assigned in a tooth numbering method according to an embodiment of the present invention. FIG. 10 is a drawing showing an example of a number that needs correction in a tooth numbering method according to an embodiment of the present invention, and FIG. 11 is a drawing showing an example of a modified number in a tooth numbering method according to an embodiment of the present invention.

Referring to FIG. 7, a tooth segmentation apparatus, for example, a tooth number designation unit of the tooth segmentation apparatus receives a segmented tooth region, for example, a tooth mesh from a tooth segmentation unit at S710. The tooth number designation unit provides an interface for designating a tooth number to a user while displaying the segmented tooth region, and receives an input of designating a tooth number from the user at S720. In some embodiments, the user may designate the number of an arbitrary tooth among a plurality of teeth. In some embodiments, as shown in FIG. 8, the user may designate the number of one tooth 810 among the teeth. In some embodiments, the tooth 810 on which the user designates the number may be a mesial tooth.

The tooth number designation unit automatically numbers the remaining teeth based on the tooth number designated by the user at S730. In some embodiments, as shown in FIG. 9, the tooth numbering unit tooth may designate the numbers in the distal direction in ascending or descending order. In some embodiments, the tooth numbers may be assigned according to a clinically used numbering system. The clinically used numbering system may be, for example, a federation dentaire internationale (FDI) system, a universal numbering system, or a Palmer notation method.

In the example shown in FIG. 8 and FIG. 9, when the user designates the number of the tooth 810 as 21, numbers of 22, 23, 24, 25, 26, and 27 may be assigned to teeth in the clockwise direction from the tooth 810, for example, according to the FDI system. Further, numbers of 11, 12, 13, 14, 15, and 16 may be assigned to teeth in a counterclockwise direction from the tooth 810 according to the FDI system.

Next, the tooth number designation unit provides an interface for checking the tooth numbers at S740, and may, when there is a tooth that needs to be corrected because the number is incorrectly assigned at S750, receive a user's operation for correcting the tooth number through the interface at S750,

The tooth number designation unit may automatically number the teeth in the distal direction again based on the tooth whose number has been corrected based on the user's operation at S730. The user may check whether the number assigned to each tooth is incorrect through the interface. As shown in FIG. 10, when there are the teeth 1010, 1020, and 1030 to which the numbers are incorrectly assigned, the user may input an operation to correct the number of the first tooth 1010 among the teeth 1010, 1020, and 1030 to which the numbers are incorrectly assigned. For example, although numbers of 15, 16, and 17 should be assigned to the teeth 1010, 1020, and 1030, respectively, numbers of 14, 15, and 16 may be assigned to the teeth 1010, 1020, and 1030, respectively. In this case, the user may input an operation correct the number of the tooth 1010 to 14. Accordingly, as shown in FIG. 11, the teeth 1020 and 1030 requiring the correction according to the numbering system may be selected from the teeth, and the numbers of the teeth 1020 and 1030 may be automatically corrected. That is, in the counterclockwise direction, the teeth 1020 and 1030 may be assigned 15 and 16, respectively.

When there is no tooth to which the number is incorrectly assigned at S750, the tooth number designation unit ends the operation of the tooth number designation. In some embodiments, when there is no tooth to which the number is incorrectly assigned at S750, the tooth number designation unit may receive an input of the user's operation to confirm that there is no tooth to which the number is incorrectly assigned.

According to the above-described embodiments, when the user inputs the number of a certain tooth, the remaining tooth number can be automatically assigned. In addition, providing the interface capable of correcting the incorrectly assigned tooth number can prevent the tooth number from being incorrectly designated.

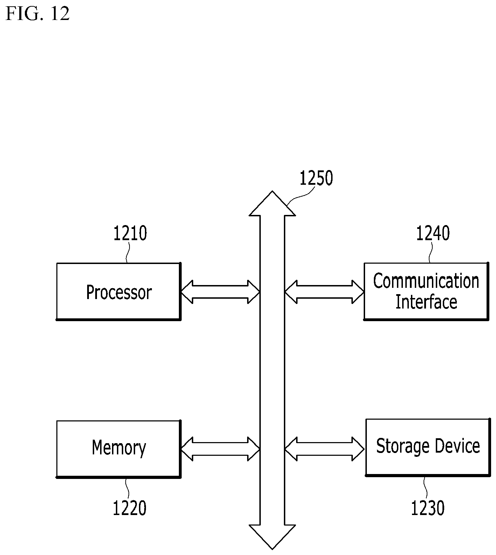

Hereinafter, an example computing device for implementing a tooth segmentation apparatus or a tooth segmentation method according to various embodiment of the present invention is described with reference to FIG. 12.

FIG. 12 is a drawing showing an example of a computing device according to an embodiment of the present invention.

Referring to FIG. 12, a computing device 1200 includes a processor 1210, a memory 1220, a storage device 1230, a communication interface 1240, and a bus 1250. The computing device 1200 may further include other general components.

The processor 1210 controls an overall operation of each component of the computing device 1200. The processor 1210 may be implemented with at least one of various processing units such as a central processing unit (CPU), an application processor (AP), a microprocessor unit (MPU), a micro controller unit (MCU), and a graphic processing unit (GPU), or may be implemented with parallel processing units. In addition, the processor 1210 may perform operations on a program for executing the above-described tooth segmentation method.

The memory 1220 stores various data, instructions, and/or information. The memory 1220 may load a computer program from the storage device 1230 to execute the above-described tooth segmentation method. The storage device 1230 may non-temporarily store a program. The storage device 1230 may be implemented as a nonvolatile memory.

The communication interface 1240 supports wired or wireless Internet communication of the computing device. In addition, the communication interface 1240 may support various communication methods other than Internet communication.

The bus 1250 provides a communication function between the components of the computing device. The bus 1250 may be implemented as various types of buses, such as an address bus, a data bus, and a control bus.

The computer program may include instructions for causing the processor 1210 to execute the tooth segmentation method when loaded into the memory 1220. That is, the processor 1210 may perform the tooth segmentation method by executing the instructions.

In some embodiments, the computer program may include instructions of receiving dental scan data in a form of a mesh, detecting a boundary box of each tooth from the dental scan data, setting a boundary condition from the boundary box of each tooth, and segmenting a tooth region of each tooth in the dental scan data based on the boundary condition of each tooth.

In some embodiments, the computer program may include instructions of generating a plurality of tooth regions of a plurality of teeth by segmenting the tooth region of each tooth in the dental scan data, displaying the plurality of tooth regions, receiving a designation for a number of at least one tooth among the plurality of teeth from a user, and automatically numbering remaining teeth among the plurality of teeth based on the number of the at least one tooth.

The tooth segmentation method according to various embodiments may be implemented as a computer-readable program on a computer-readable medium. In one embodiment, the computer-readable medium may include a removable recording medium or a fixed recording medium. In another embodiment, the computer-readable program recorded on the computer-readable medium may be transmitted to another computing device via a network such as the Internet and installed in another computing device, so that the computer program can be executed by another computing device.

While this invention has been described in connection with what is presently considered to be practical embodiments, it is to be understood that the invention is not limited to the disclosed embodiments. On the contrary, it is intended to cover various modifications and equivalent arrangements included within the spirit and scope of the appended claims.

## Claims

1. A tooth segmentation apparatus comprising:
a memory configured to store one or more instructions; and
a processor configured to, by executing the one or more instructions:
detect a boundary box of each tooth from dental scan data in a form of a mesh;
set a boundary condition from the boundary box of each tooth; and
segment a tooth region of each tooth in the dental scan data based on the boundary condition of each tooth.

2. The tooth segmentation apparatus of claim 1, wherein the processor is configured to detect the bounding box of each tooth from the dental scan data based on an object detection model of an artificial neural network.

3. The tooth segmentation apparatus of claim 1, wherein the processor is configured to:
derive a first vertex of the mesh corresponding to a center of the bounding box and a plurality of second vertices of the mesh respectively corresponding to a plurality of vertices of the bounding box;
find a length of a predetermined shorted path among a plurality of shortest paths from each of the plurality of second vertices to the first vertex; and
assign values of the boundary condition to a plurality of third vertices of the mesh corresponding to the length of the predetermined shortest path from the first vertex.

4. The tooth segmentation apparatus of claim 3, wherein the predetermined shortest path is a longest shortest path among the plurality of shortest paths.

5. The tooth segmentation apparatus of claim 3, wherein the processor is configured to segment the tooth region based on a Laplace equation using the values of the boundary condition as an input.

6. The tooth segmentation apparatus of claim 1, wherein the processor is configured to:
generate a plurality of tooth regions of a plurality of teeth by segmenting the tooth region of each tooth in the dental scan data;
provide an interface configured to check segmentation of the tooth region of each tooth; and
receive an operation for correction of a tooth region requiring correction among the plurality of tooth regions.

7. The tooth segmentation apparatus of claim 6, wherein the processor is configured to:
assign a corresponding color to the tooth region of each tooth; and
receive the operation for correction through coloring of the tooth region requiring correction.

8. The tooth segmentation apparatus of claim 1, wherein the processor is configured to:
generate a plurality of tooth regions of a plurality of teeth by segmenting the tooth region of each tooth in the dental scan data;
display the plurality of tooth regions;
receive a designation for a number of at least one tooth among the plurality of teeth from a user; and
automatically numbering remaining teeth among the plurality of teeth based on the number of the at least one tooth.

9. The tooth segmentation apparatus of claim 8, wherein the processor is configured to automatically number the remaining teeth according to a clinically used numbering system.

10. The tooth segmentation apparatus of claim 8, wherein the processor is configured to:
provide an interface configured to check a number of each tooth;
receive an operation for number correction of a tooth whose number requires correction among the plurality of teeth through the interface; and
automatically correct a number of a portion of the plurality of teeth based on the number correction.

11. The tooth segmentation apparatus of claim 10, wherein the processor is configured to select the portion of plurality of teeth selected according to a clinically used numbering system.

12. A tooth segmentation method performed by a computing device, the method comprising:
receiving dental scan data in a form of a mesh;
detecting a boundary box of each tooth from the dental scan data;
setting a boundary condition from the boundary box of each tooth; and
segmenting a tooth region of each tooth in the dental scan data based on the boundary condition of each tooth.

13. The method of claim 12, wherein detecting the bounding box comprises detecting the bounding box of each tooth from the dental scan data based on an object detection model of an artificial neural network.

14. The method of claim 12, further comprising:
generating a plurality of tooth regions of a plurality of teeth by segmenting the tooth region of each tooth in the dental scan data;
displaying the plurality of tooth regions;
receiving a designation for a number of at least one tooth among the plurality of teeth from a user; and
automatically numbering remaining teeth among the plurality of teeth based on the number of the at least one tooth.

15. A computer program executed by a computing device and stored in a non-transitory recording medium, the computer program causing the computing device to execute:
receiving dental scan data in a form of a mesh;
detecting a boundary box of each tooth from the dental scan data;
setting a boundary condition from the boundary box of each tooth; and
segmenting a tooth region of each tooth in the dental scan data based on the boundary condition of each tooth
